(19) Europäisches Patentamt
European Patent Office
Office européen des brevets

(11) Veröffentlichungsnummer: **0 045 423**
**B1**

(12) # EUROPÄISCHE PATENTSCHRIFT

(45) Veröffentlichungstag der Patentschrift: **22.02.84**

(21) Anmeldenummer: **81105672.0**

(22) Anmeldetag: **20.07.81**

(51) Int. Cl.³: **C 07 C 17/14,** C 07 B 9/00, C 07 C 21/24, C 07 C 79/12, C 07 C 79/36, C 07 C 79/46

(54) **Verfahren zur alpha-Halogenierung von gegebenenfalls substituierten Methylaromaten.**

(30) Priorität: **01.08.80 DE 3029369**

(43) Veröffentlichungstag der Anmeldung: **10.02.82 Patentblatt 82/6**

(45) Bekanntmachung des Hinweises auf die Patenterteilung: **22.02.84 Patentblatt 84/8**

(84) Benannte Vertragsstaaten: **BE CH DE FR GB IT LI**

(56) Entgegenhaltungen:
**EP - A - 0 001 281**
**EP - A - 0 045 425**

**Chemical Abstracts Band 54, Nr. 15, 1960 Columbus, Ohio, USA R.E. BUCKLES et al. "Addition reactions of mixtures of bromine and chlorine" Spalte 15304b-h**
**JOURNAL OF ORGANIC CHEMISTRY, Band 43, Nr. 14, 1978 Columbus G.E. HEASLEY et al. "Electrophilic additions to Dienes and the 1-Phenylpropenes with Pyridine-Halogen Complexes and Tribromides. Effects on Stereochemistry and Product Ratios"**
**Patents Abstracts of Japan Band 3, Nr. 72, 21. Juni 1979 Seite 18C49**
**Louis F. Fieser and Mary Fieser, Organic Chemistry, 3rd ed (1956), S. 566-567**

(73) Patentinhaber: **BAYER AG, Zentralbereich Patente, Marken und Lizenzen, D-5090 Leverkusen 1, Bayerwerk (DE)**

(72) Erfinder: **Blank, Heinz Ulrich, Dr., Am Geusfelde 35, D-5068 Odenthal (DE)**
Erfinder: **Wolters, Erich, Dr., Streffenweg 22, D-5162 Niederzier (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 045 423 B1

BUNDESDRUCKEREI BERLIN

# Verfahren zur α-Halogenierung von gegebenenfalls substituierten Methylaromaten

Die vorliegende Erfindung betrifft ein Verfahren zur α-Halogenierung von gegebenenfalls substituierten Methylaromaten mit Chlorbrom oder Chlorbrom enthaltenden Gemischen von Halogenierungsmitteln.

Es ist bekannt, Alkylaromaten mit Chlor oder mit Brom in der α-Stellung zu halogenieren. Im allgemeinen geht man davon aus, daß die α-Bromierung mit sehr viel höheren Selektivitäten zu Benzylhalogeniden und Benzalhalogeniden führt als die α-Chlorierung. Andererseits ist das Brom als Chemikalie sehr viel teurer als das Chlor. Dieser Kostennachteil schlägt immer dann voll durch, wenn nicht die Halogenierungsprodukte mit dem speziellen Halogen gewünscht werden, sondern deren Verseifungsprodukte, beispielsweise zu den entsprechenden Benzylalkoholen oder den entsprechenden Benzaldehyden, wobei das Halogen, insbesondere das teure Brom, vollständig als Halogenwasserstoff oder als Salz des Halogenwasserstoffs, insbesondere als HBr oder als Bromid abgespalten werden und zur Wiedergewinnung des Broms kostenaufwendig isoliert und auf freies Brom aufgearbeitet werden müssen.

Beispielsweise ist aus der DE-OS 28 49 692 bekannt, p-tert.-Butylbenzalbromid dadurch herzustellen, daß man p-tert.-Butyltoluol mit 2 Mol Brom pro Mol des Butyltoluols bei einer Temperatur von 100 bis 110°C unter Einwirkung von Ultraviolett-Bestrahlung umsetzt. Diese DE-OS enthält auf Seite 4 zur radikalischen Chlorierung den Hinweis, daß beispielsweise bei der Chlorierung von tert.-Butyltoluol nicht nur das erwünschte tert.-Butylbenzalchlorid gebildet wird, sondern daß dabei in beträchtlichem Maße kernchlorierte Produkte erhalten werden, was aus dem Befund gefolgert wird, daß das eingeführte Chlor nur teilweise durch alkalische Hydrolyse wieder abgespalten werden kann.

Es wurde nun ein Verfahren zur α-Halogenierung von substituierten Methylaromaten gefunden, das dadurch gekennzeichnet ist, daß man Methylaromaten der allgemeinen Formel

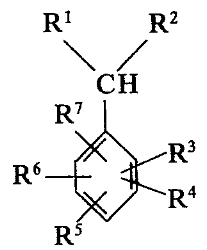

(I)

in der

$R^1$ und $R^2$ unabhängig voneinander Wasserstoff, Chlor oder Brom bedeuten,

$R^3$ für Wasserstoff, Halogen, Nitro, die Sulfonsäuregruppe, Sulfonylhalogenid, die Carboxylgruppe, Alkoxycarbonyl, Aryloxycarbonyl, Carbonylhalogenid, die $CHR^1R^2$-Gruppe, wobei $R^1$ und $R^2$ die angegebene Bedeutung haben, Cyano, Isocyanato, Cyanato, Isothiocyanato, Thiocyanato, Aryl, Aryloxy, Arylthio, Aryldithio, tert.-Alkyl oder Alkyloxy steht,

$R^4$ für Wasserstoff, Halogen, Nitro, die Sulfonsäuregruppe, Sulfonylhalogenid, die Carboxylgruppe, Alkoxycarbonyl, Aryloxycarbonyl, Carbonylhalogenid oder die $CHR^1R^2$-Gruppe steht,
worin $R^1$ und $R^2$ die gegebene Bedeutung haben, und

$R^5$ bis $R^7$ unabhängig voneinander Wasserstoff oder Halogen bedeuten,

wobei zusätzlich zwei der Reste $R^4$, $R^5$, $R^6$ und $R^7$, wenn sie benachbart sind, Teile eines kondensierten aromatischen oder cycloaliphatischen Ringes sein können und
wobei weiterhin die Arylteile der genannten Substitutenten ihrerseits substituiert sein können, mit Ausnahme von gegebenenfalls substituierten 4-tert.-Butyltoluolen, deren α-Halogenierung Gegenstand der EP-Patentanmeldung Nr. 0 045 425 ist, bei einer Temperatur von 40 bis 250°C mit Chlorbrom oder Chlorbrom enthaltenden Halogenierungsmitteln umsetzt, wobei gegebenenfalls in Gegenwart von Radikalbildnern und/oder bei Bestrahlung mit Licht sowie gegebenenfalls in Gegenwart von Lösungsmitteln gearbeitet wird.

Als Halogen sei beispielsweise Fluor, Chlor, Brom oder Jod, bevorzugt Fluor, Chlor oder Brom, besonders bevorzugt Chlor oder Brom, genannt.

Als Sulfonylhalogenid sei beispielsweise Sulfonylfluorid, Sulfonylchlorid oder Sulfonylbromid, bevorzugt Sulfonylfluorid oder Sulfonylchlorid, besonders bevorzugt Sulfonylchlorid, genannt.

Als Alkoxycarbonyl seien beispielsweise Estergruppen genannt, die einschließlich der Carbonylgruppe 2 bis 13, bevorzugt 2 bis 5, besonders bevorzugt 2 bis 3 Kohlenstoffatome enthalten, wie Methoxycarbonyl, Ethoxycarbonyl, Propoxycarbonyl, Butyloxycarbonyl, Hexyloxycarbonyl, Octyloxycarbonyl oder Dodecyloxycarbonyl, wobei die Kohlenwasserstoffkette geradkettig oder verzweigt

sein kann.

Als Aryloxycarbonyl seien beispielsweise Estergruppen genannt, die als phenolische Komponente beispielsweise Phenol, Naphthol oder Anthranol, bevorzugt Phenol enthalten.

Als Carbonylhalogenid sei beispielsweise Carbonylfluorid, Carbonylchlorid oder Carbonylbromid, bevorzugt Carbonylchlorid, genannt.

Als Aryl seien aromatische Reste, wie Phenyl, Naphthyl, Anthryl, Biphenyl, Phenanthryl, Benzylphenyl, Phenoxyphenyl, bevorzugt Phenyl, genannt.

Als Aryloxy seien Reste genannt, die von einer phenolischen Verbindung abgeleitet sind, wie Phenoxy, Naphthyloxy, Anthryloxy, Biphenyloxy, bevorzugt Phenyloxy.

Als Arylthio seien beispielsweise die Thioanaloga der beschriebenen Aryloxyreste genannt. Bevorzugt ist unter den Arylthioresten der Phenylthiorest.

Als Aryldithioreste seien beispielsweise die Dithioanaloga der oben beschriebenen Aryloxyreste genannt. Bevorzugt ist der Dithio-phenylrest, also der über eine Disulfidbrücke gebundene Phenylrest.

Als tertiäres Alkyl seien verzweigte Kohlenwasserstoffreste genannt, deren $\alpha$-C-Atom ein tertiäres C-Atom ist und die beispielsweise 4 bis 8 C-Atome, bevorzugt 5 bis 8 C-Atome, haben, wie tert.-Butyl, tert.-Amyl, tert.-Hexyl oder tert.-Octyl.

Als Alkyloxy seien Reste genannt, die sich von einem aliphatischen geradkettigen oder verzweigten Alkohol mit beispielsweise 1 bis 12, bevorzugt 1 bis 4, besonders bevorzugt 1 bis 2, C-Atomen ableiten, wie Methoxy, Ethoxy, Propoxy, Butyloxy, Hexyloxy, Octyloxy oder Dodecyloxy.

Weiterhin können zwei der Reste $R^4$, $R^5$, $R^6$ und $R^7$, wenn sie benachbart sind, Teile eines kondensierten aromatischen oder cycloaliphatischen Ringes sein. Hierdurch gelangt man unter Einbeziehung des in Formel (I) bereits dargestellten Benzolkernes beispielsweise zum Naphthalinsystem, zum Indansystem oder zum Tetrahydronaphthalinsystem.

Selbstverständlich können die in den Substituenten enthaltenen Arylteile ihrerseits weiter substituiert sein. Für solche weitere Substitution sei beispielsweise Halogen, Nitro, Sulfonylhalogenid, Carbonylhalogenid, Aryloxy, oder Alkyl, bevorzugt Halogen oder Nitro, genannt.

Für den Fall, daß zwei Reste die Carboxylgruppe bedeuten und benachbart sind, kann an Stelle der beiden Carboxylgruppen auch die daraus entstandene Anhydridgruppe stehen.

An Stelle eines carbocyclischen Aromaten kann auch ein Heteroaromat treten, der durch eines oder mehrere N-, S- oder O-Atome im Ring charakterisiert ist, 5 oder 6 Ringglieder hat, mit einem Benzolkern oder einem weiteren Heterocyclus kondensiert ist und mindestens eine $CHR^1R^2$-Gruppe sowie gegebenenfalls Substituenten der genannten Art trägt.

Als Verbindungen der allgemeinen Formel (I) können in das erfindungsgemäße Verfahren beispielsweise eingesetzt werden Toluol, o-Xylol, m-Xylol, p-Xylol (Produkte sind beispielsweise Benzylhalogenid, Benzalhalogenide, $\alpha,\alpha$-Dihalogenxylol, $\alpha,\alpha$-Trihalogenxylol, $\alpha,\alpha$-Tetrahalogenxylol, $\alpha,\alpha$-Pentahalogenxylol, $\alpha,\alpha$-Hexahalogenxylol), o-Chlortoluol (Reaktionsprodukte o-Chlorbenzylhalogenid, o-Chlorbenzalhalogenid, o-Chlorbenzotrihalogenid), p-Chlortoluol (Produkte p-Chlorbenzylhalogenid, p-Chlorbenzalhalogenid, p-Chlorbenzotrihalogenid), m-Chlortoluol (Produkte m-Chlorbenzylhalogenid, m-Chlorbenzalhalogenid, m-Chlorbenzotrihalogenid), p-Nitrotoluol (Produkte p-Nitrobenzylhalogenid, p-Nitrobenzalhalogenid, p-Nitrobenzotrihalogenid), 2,4-Dichlortoluol (Reaktionsprodukte beispielsweise 2,4-Dichlorbenzalhalogenid, 2,4-Dichlorbenzotrihalogenid), 2,4,5,6-Tetrachlor-m-xylol, Diphenylchlormethan, Mesitylen, Pseudocumol, 2,3,4,5,6-Pentachlortoluol, 3,4,5,6-Tetrachlortoluol, 3,4,5,6-Tetrachlor-1,2-dimethylbenzol, 2,4,5,6-Tetrachlor-1,3-dimethylbenzol, 2,3,5,6-Tetrachlor-1,4-dimethylbenzol, 2,4,6-Trichlor-1,3,5-trimethylbenzol, 5,6,7,8-Tetrachlortetralin, 1-Methylnaphthalin, 2-Methylnapthalin, 2,3-Dichlortoluol, 2,5-Dichlortoluol, 2,6-Dichlortoluol, 3,4-Dichlortoluol, 3,5-Dichlortoluol, 2-Bromtoluol, 3-Bromtoluol, 4-Bromtoluol, 2-Fluortoluol, 3-Fluortoluol, 4-Fluortoluol, Fluortoluole, wie beispielsweise 4-Fluor-3-chlortoluol, 4-Fluor-2,5-dichlortoluol, Fluor-bromtoluole, wie beispielsweise 4-Fluor-3-bromtoluol, 4-Fluor-2,5-dibromtoluol, 4-Fluor-2,6-dibromtoluol, Phenoxytoluole, wie beispielsweise 2-Phenoxytoluol, 3-Phenoxytoluol, 4-Phenoxytoluol, 4-Fluor-3-phenoxytoluol, 4-Chlor-3-phenoxytoluol, 4-Brom-3-phenoxytoluol, 4-Fluor-3-(4-fluorphenoxy)-toluol, 4-Fluor-(3,4-bromphenoxy)-toluol, 3-tert.-Butyltoluol, 4-tert.-Butyltoluol, p-Toluolsulfonsäure, p-Toluolsulfonylchlorid, p-Toluolsulfonylfluorid, 3-Nitrotoluol, 3-Nitrobenzylhalogenid, 4-Nitrobenzylhalogenid, m-Toloylisocyanat, p-Toloylisocyanat, Toluol-2,4-diisocyanat, Toluol-2,6-diisocyanat, p-Toluol-sulfonsäure, p-Toluolsulfonylchlorid, 2-Chlorbenzylsulfonsäurechlorid, 3-Methylphthalsäureanhydrid, 4-Methylphthalsäureanhydrid, Benzylchlorid, Benzalchlorid, o-Chlorbenzylchlorid, o-Chlorbenzalchlorid, p-Chlorbenzylchlorid, 2,4-Dichlorbenzylchlorid, 3,4-Dichlorbenzylchlorid, 2,3-Dichlorbenzylchlorid, 2,5-Dichlorbenzylchlorid, Trichlorbenzylchlorid, 2-Chlor-4-nitrotoluol, 2,4-Dinitritoluol, 2,6-Dinitrotoluol, 2-Nitro-1,3-dimethylbenzol, 4-Nitro-1,3-dimethylbenzol, 2-Nitro-1,4-dimethylbenzol, o-Nitrotoluol, p-Nitrosulfonsäure, o-Nitrotoluol-p-sulfonsäurechlorid, p-Nitrotoluol-o-sulfonsäure, p-Nitrotoluol-o-sulfonsäurechlorid, 2,2'-Dimethyldiphenylether, 3,2'-Dimethyldiphenylether, 4,2'-Dimethyldiphenylether, 3,3'-Dimethyldiphenylether, 4,3'-Dimethyldiphenylether, 4,4'-Dimethyldiphenylether, 2-Methoxytoluol, 3-Methoxytoluol, 2-Chlor-5-methoxytoluol, 2-Methoxy-4-nitrotoluol, 4-Methoxy-3,5-dinitrotoluol, 3-Chlor-6-methoxytoluol, 3-Benzyl-4-methoxychlortoluol, 3-Methoxy-6-nitrotoluol, 4-Methoxy-3-nitrotoluol, 2,4-Dibromtoluol, 2,5-Dibromtoluol, 2,6-Dibromtoluol, 3,4-Dibromtoluol, 2,3,4-Tribromtoluol, 2,4,5-Tribromtoluol, m-Toluylisocyanat, 3,5-Dimethylphenylisocyanat, p-Toluolisocyanat, o-Toluylisocyanat, 3-Chlor-4-methylphenylisocyanat, o-Toluylsäure, o-Methylbenzoylchlorid, 3,5-Dinitro-o-toluylsäure, 3,5-Dinitro-o-toluylchlorid, m-Toluylsäure, m-Methylbenzoylchlorid, 3-Methyl-6-nitrobenzoylchlorid, 3-Methyl-4-nitrobenzoylchlorid, p-Methyl-benzoylchlorid, 4-Methyl-3-nitrobenzoesäure, 4-Methyl-3-nitrobenzoylchlorid, p-Toluylsäure, 3-Nitro-4-methylbenzamid.

Weiterhin ist es möglich, heterocyclische Verbindungen in das beanspruchte Verfahren einzusetzen, beispielsweise 2-Methyl-, 3-Methyl-, 4-Methylpyridin, 5-Methyl- und 6-Methylchinoxalin, 2,3-Dichlor-5-methyl-chinoxalin, 2,3-Dichlor-6-methylchinoxalin, 2,3-Dichlor-5-methylchinoxalin, 2,3-Dichlor-6-methylchinoxalin, 2-Methylbenzoxazol, 2,4,5-Trichlor-6-methylpyrimidin, 2,4-Dichlor-6-methylpyrimidin, 2,4-Dichlor-6-pyrimidincarbonsäurechlorid.

Von den im erfindungsgemäßen Verfahren einsetzbaren substituierten Methylaromaten der Formel (I) seien bevorzugt solche der Formel

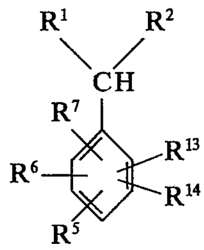

(II)

genannt, in der

$R^1$, $R^2$, $R^5$, $R^6$ und $R^7$ die genannte Bedeutung haben,
$R^{13}$ für Wasserstoff, Halogen, Nitro, die Sulfonsäuregruppe, Sulfonylhalogenid, Alkoxycarbonyl, Phenoxycarbonyl, Carbonylhalogenid, die $CHR^1R^2$-Gruppe, in der $R^1$ und $R^2$ die genannte Bedeutung haben, Phenyl, Phenyloxy, Phenylthio, Phenyldithio, tert.-Alkyl oder Alkoxy steht und
$R^{14}$ Wasserstoff, Fluor, Chlor, Brom oder Nitro bedeutet.

Unter den im erfindungsgemäßen Verfahren einsetzbaren substituierten Methylaromaten der Formel (II) seien bevorzugt solche der Formel

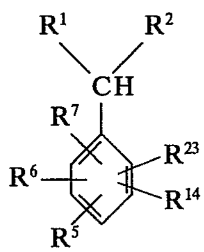

(III)

genannt, in der

$R^1$, $R^2$, $R^5$, $R^6$, $R^7$ und $R^{14}$ die genannte Bedeutung besitzen und
$R^{23}$ für Wasserstoff, Halogen, Nitro, die Sulfonsäuregruppe, Sulfonylhalogenid, Alkoxycarbonyl oder Carbonylhalogenid steht.

Unter den Verbindungen der Formel (III) für das erfindungsgemäße Verfahren seien bevorzugt solche der Formel

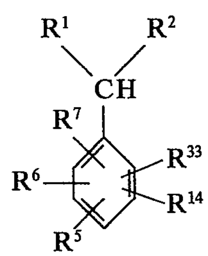

(IV)

genannt, in der

$R^1$, $R^2$, $R^5$, $R^6$, $R^7$ und $R^{14}$ die genannte Bedeutung haben und
$R^{33}$ für Wasserstoff, Fluor, Chlor, Sulfonylfluorid oder Sulfonylchlorid steht.

Unter den Verbindungen der Formel (IV) für das erfindungsgemäße Verfahren seien bevorzugt substituierte Nitro-methylaromaten der Formel

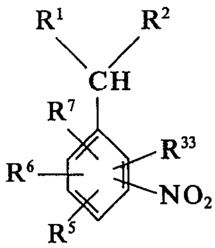

(V)

genannt, in der

$R^1$, $R^2$, $R^5$, $R^6$, $R^7$ und $R^{33}$ die genannte Bedeutung besitzen.

Weiterhin werden in bevorzugter Weise für das erfindungsgemäße Verfahren solche substituierten Methylaromaten eingesetzt, die eine nicht substituierte Methylgruppe tragen. Solche bevorzugten Verbindungen können jeweils aus den Formel (I), (II), (III), (IV) und (V) abgeleitet werden und durch die Formeln

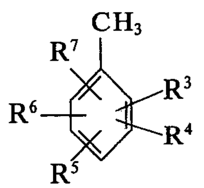

(VI)

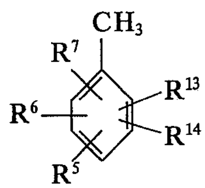

(VII)

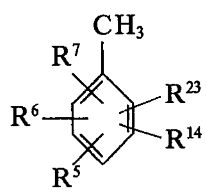

(VIII)

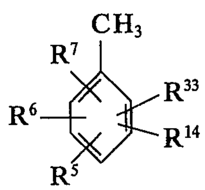

(IX)

und

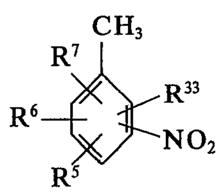

(X)

dargestellt werden, in denen die jeweiligen Substituenten die obengenannte Bedeutung besitzen.

Von den Verbindungen der Formel (VI) werden in bevorzugter Weise im erfindungsgemäßen Verfahren solche der Formel

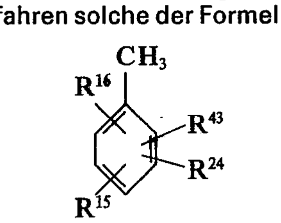

(XI)

eingesetzt, in der

$R^{15}$ und $R^{16}$ unabhängig voneinander für Wasserstoff, Fluor, Chlor oder Brom stehen,
$R^{24}$ Nitro, Fluor, Chlor oder Brom bedeutet und
$R^{43}$ Wasserstoff, Nitro oder Aryloxy bedeutet.

Unter diesen Verbindungen der Formel (XI) sind wiederum für das erfindungsgemäße Verfahren solche der Formel

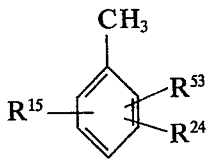

(XII)

bevorzugt, in der

$R^{15}$ und $R^{24}$ die angegebene Bedeutung haben und
$R^{53}$ für Wasserstoff oder Phenoxy steht.

In besonders bevorzugter Weise wird im erfindungsgemäßen Verfahren das p-$NO_2$-Toluol eingesetzt.
Weitere bevorzugte Verbindungen der Formel (XI) sind solche der Formel

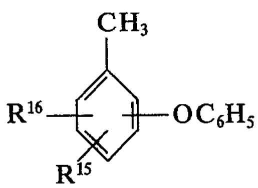

(XIII)

in der

$R^{15}$ und $R^{16}$ die angegebene Bedeutung haben.

Besonders bevorzugt werden solche Verbindungen der Formel (XIII) im erfindungsgemäßen Verfahren eingesetzt, in denen die Phenoxygruppe in meta-Stellung zur Methylgruppe steht.

Die $\alpha$-Halogenierung von substituierten Methylaromaten wird erfindungsgemäß durch Umsetzung mit Chlorbrom als Halogenierungsmittel durchgeführt. Das als Halogenierungsmittel verwendete Chlorbrom, auch als Bromchlor oder Bromchlorid bekannt, kann in Gegenwart von Brom und/oder Chlor verwendet werden. Das Halogenierungsmittel wird beispielsweise in gasförmiger oder flüssiger Form oder in gelöster Form in üblicher Weise über eine Dosiereinrichtung in das Reaktionsgemisch eingebracht, wobei die Dosierung bevorzugt in dem Maße erfolgt, wie das Halogenie-ungsmittel im Reaktionsansatz verbraucht wird, so daß gewöhnlich bei einer stationären Konzentration an Halogenierungsmittel von nicht mehr als 5 Gew.-%, bevorzugt nicht mehr als 2 Gew.-%, besonders bevorzugt nicht mehr als 1 Gew.-%, bezogen auf das gesamte Reaktionsgemisch gearbeitet wird. Für den Fall, daß das Halogenierungsmittel gasförmig in den Reaktor eingebracht wird, wird es in geeigneter Weise vor dem Reaktor verdampft, wobei gegebenenfalls ein Intertgas zugemischt wird. Als Inertgase seien beispielsweise Stickstoff, Chlorwasserstoff, Bromwasserstoff, Kohlendioxid oder Schwefeldioxid genannt. Vorzugsweise verwendet man Stickstoff, Chlorwasserstoff oder Bromwasserstoff. Es können auch mehrere der genannten Inertgase gleichzeitig verwendet werden. Das Halogenierungsmittel wird gewöhnlich durch Vermischen von Brom mit Chlor und/oder durch Vermischen von Bromwasserstoff mit Chlor hergestellt. Beispielsweise vermischt man Brom und/oder Bromwasserstoff mit Chlor im Verhältnis von 10 Mol Brom zu 1 Mol Chlor bis 1 Mol Brom zu 10 Mol Chlor, wobei anstelle von Brom auch die äquivalente Menge Bromwasserstoff benutzt werden kann. Vorzugsweise enthält das Halogenierungsmittel Brom und Chlor im Molverhältnis 2 zu 1 bis 1 zu 2 und insbeosndere im Verhältnis von etwa 1 zu 1. Das Halogenierungsmittel kann in der Flüssig- oder Gasphase hergestellt werden, gegebenenfalls in Gegenwart eines gasförmigen oder flüssigen Verdünnungsmittels. Ein derart hergestelltes gasförmiges bromchlorhaltiges Halogenierungsmittel kann direkt für das erfindungsgemäße Verfahren verwendet werden. Es ist jedoch auch möglich, ein solches Halogenierungsmittel durch Kühlung zu kondensieren oder in einem Lösungsmittel zu lösen. Ebenso können entsprechend hergestellte Bromchlor enthaltende Halogenierungsmittel, die in der Flüssigphase, gegebenenfalls in Gegenwart eines Lösungsmittels, hergestellt wurden, direkt für das erfindungsgemäße Verfahren verwendet werden.

Als Lösungsmittel, die gegebenenfalls zur Aufnahme des Halogenierungsmittels oder als

Reaktionsmedium für das erfindungsgemäße Verfahren eingesetzt werden können, werden solche verwendet die unter den Bedingungen des erfindungsgemäßen Verfahrens nicht verändert werden.

Als geeignete Lösungsmittel seien beispielsweise genannt: halogenierte aliphatische Kohlenwasserstoffe mit 1 bis 5 Kohlenstoffatomen, bevorzugt 1 bis 2 Kohlenstoffatomen wie Ethylenchlorid, Chloroform, Tetrachlorkohlenstoff, Dichlorethan, Trichlorethan, Tetrachlorethan und Hexachlorethan, aromatische Kohlenwasserstoffe und deren Halogenderivate wie Benzol, Brombenzol, Chlorbenzol, Dichlorbenzol, Trichlorbenzol oder Tetrachlorbenzol, sowie Schwefelkohlenstoff. In bevorzugter Weise werden Tetrachlorkohlenstoff oder chlorierte Benzole verwendet. Hierbei ist die Verwendung von Tetrachlorkohlenstoff in einem Temperaturbereich von beispielsweise unter 100° C bevorzugt, die Verwendung chlorierter Benzole in einem Bereich von 100° C oder darüber. Für den Fall, daß oberhalb des Siedepunktes eines einzusetzenden Lösungsmittels gearbeitet werden soll, kann hierbei unter einem solchen Überdruck gearbeitet werden, bei dem das Lösungsmittel als Flüssigphase vorliegt. Selbstverständlich können außer den reinen Lösungsmitteln auch Gemisch der genannten Lösungsmittel untereinander eingesetzt werden.

Die Menge des einzusetzenden Lösungsmittels ist in weiten Grenzen variabel. Die optimale Menge kann leicht in Abhängigkeit von der Reaktionstemperatur durch Vorversuche ermittelt werden. Beispielsweise sei eine Menge von 0,1 bis zu 1000 Vol.-%, bezogen auf die Menge des Halogenierungsmittels und/oder des gesamten Reaktionsansatzes genannt. Bevorzugte Mengen sind 5 bis 500 Vol.-%, besonders bevorzugt 10 bis 100 Vol.-% Lösungsmittel bezogen auf das Halogenierungsmittel und/oder das gesamte Reaktionsgemisch.

Beispielsweise kann bei Verwendung von Tetrachlorkohlenstoff als Lösungsmittel und bei einer Temperatur oberhalb des Siedepunktes von Tetrachlorkohlenstoff die Menge des Tetrachlorkohlenstoffs mit Vorteil so gemessen werden, daß bei der gewünschten Reaktionstemperatur und bei Normaldruck gerade etwa Rückflußbedingungen vorliegen.

Die erfindungsgemäße Halogenierung verläuft unter Wärmeabgabe. Zur Einhaltung der gewünschten Reaktionstemperatur wird gewöhnlich unter gleichzeitiger Wärmeabfuhr der Reaktionswärme über einen Wärmeaustauscher, beispielsweise den Rückflußkühler für das eingesetzte Lösungsmittel, gearbeitet. Selbstverständlich kann zur Einhaltung der Reaktionstemperatur auch eine geeignete Heiz-/Kühleinrichtung am Reaktionsgefäß vorgesehen werden. Weiterhin kann für den Fall, daß der zu halogenierende substituierte Methylaromat bei der Reaktionstemperatur siedet, auch durch einen Rückfluß dieses Methylaromaten Reaktionswärme abgeführt werden. In solchen Fällen kann auf die Mitverwendung eines Lösungsmittels verzichtet werden. Solche Varianten des erfindungsgemäßen Verfahrens ohne Benutzung von Lösungsmitteln sind bevorzugt.

Das erfindungsgemäße Verfahren kann beispielsweise bei einer Temperatur von 40 bis 250° C, bevorzugt von 80 bis 220° C, besonders bevorzugt von 100 bis 290° C und ganz besonders bevorzugt von 130 bis 190° C, durchgeführt werden. Die erfindungsgemäße Halogenierung wird vorzugsweise so durchgeführt, daß die Reaktionspartner zumindest teilweise als flüssige Phase vorliegen. Wie bereits weiter oben im Zusammenhang mit leichter flüchtigen Lösungsmitteln erwähnt wurde, kann hierzu gegebenenfalls ein solcher Überdruck angewandt werden, daß auch bei Anwendung einer höheren Temperatur aus dem genannten Temperaturbereich eine solche flüssige Phase vorliegt.

Neben der bevorzugten Durchführung des erfindungsgemäßen Verfahrens in zumindest teilweiser Flüssigphase ist es jedoch grundsätzlich auch möglich, in der Gasphase zu arbeiten.

Der während der Umsetzung als Nebenprodukt entstehende Halogenwasserstoff in Form eines Gemisches aus Chlorwasserstoff und Bromwasserstoff wird vorteilhafterweise zügig aus dem Reaktionsgemisch entfernt. Die Entfernung des Halogenwasserstoffes verläuft gewöhnlich unter Normaldruck rasch genug. Sofern die Reaktion unter Druck durchgeführt werden soll, ist es sinnvoll, bei konstanten Drucken im Bereich von etwa 1 bis 10 bar zu arbeiten und gleichzeitig den bei der Reaktion anfallenden Halogenwasserstoff kontinuierlich oder absatzweise, bevorzugt kontinuierlich, aus dem Reaktionsgemisch zu entfernen.

Es ist ebenso möglich, den bei der Reaktion anfallenden Halogenwasserstoff kontinuierlich aus dem Reaktionsgemisch zu extrahieren. Hierzu kann beispielsweise die erfindungsgemäße Halogenierung in Gegenwart einer Wasserphase durchgeführt werden. Hierbei wird zweckmäßigerweise so vorgegangen, daß der in die Wasserphase übergehende Halogenwasserstoff kontinuierlich teilweise oder vollständig neutralisiert wird, wozu der pH-Wert der wäßrigen Phase im Bereich von etwa 1 bis 8, bevorzugt 2 bis 7, besonders bevorzugt 3 bis 6 eingehalten wird. Zur teilweisen oder vollständigen Neutralisierung werden beispielsweise Hydroxide, wie Natriumhydroxid, Kaliumhydroxid oder Calciumhydroxid, Hydrogencarbonate wie Natriumhydrogencarbonat, Kaliumhydrogencarbonat oder Carbonate wie Natriumcarbonat, Kaliumcarbonat, Magnesiumcarbonat oder Calciumcarbonat verwendet. Diese Neutralisationsmittel können beispielsweise in Form ihrer wäßrigen Lösungen, als Suspensionen in Wasser oder in einem der genannten organischen Lösungsmittel oder als Feststoffe in das Reaktionssystem eingebracht werden. Die Mitverwendung einer wäßrigen Phase zur Extraktion des entstehenden Halogenwasserstoffes wird bevorzugt unter Normaldruck bis zu Temperaturen angewandt, bei denen das Wasser oder ein sich etwa bildendes Azeotrop siedet. Bei Anwendung von Überdruck kann die Temperatur bei dieser Verfahrensvariante jedoch auch über 100° C liegen.

Das erfindungsgemäße Verfahren kann beispielsweise in Gegenwart von Radikalbildnern oder unter

Bestrahlung mit natürlichem oder künstlichem Licht durchgeführt werden. Das erfindungsgemäße Verfahren kann jedoch auch ohne diese genannten Hilfsmittel durchgeführt werden. Diese letztere Variante ist bevorzugt, da hierbei der technische Aufwand geringer ist und weiterhin keinerlei Reste des etwa verwendeten Radikalbildners im Reaktionsgemisch verbleiben. Diese Verfahrensvariante ohne photochemische oder durch Radikalbildner hervorgerufene Initiierung kann weiterhin in einer besonders bevorzugten Variante auch ohne Lösungs- oder Verdünnungsmittel sowohl für das zugefügte Halogenierungsmittel als auch für das gesamte Reaktionsgemisch gearbeitet werden. In bestimmten Fällen, beispielsweise bei der α-Halogenierung von thermisch labilen Substanzen, bei der im Temperaturbereich von unter etwa 100° C gearbeitet werden soll, kann es jedoch zweckmäßig sein, das Reaktionsgemisch gleichzeitig zu bestrahlen und/oder Radikalbildner zu verwenden. In diesen Fällen wird vorzugsweise in Gegenwart eines Verdünnungsmittels, beispielsweise Tetrachlorkohlenstoff, gearbeitet. Die Bestrahlung erfolgt beispielsweise mit den üblicherweise auch für Chlorierungsreaktionen verwendeten Lichtquellen, wie Glühlampen, Fluoreszenzlampen oder Quecksilberdampflampen. Als Radikalbildner können geringe Mengen an organischen Peroxiden oder an aliphatischen Diazoverbindungen verwendet werden, wie Azoisobuttersäuredinitril und Hydrazoisobuttersäuredinitril.

Die erfindungsgemäße Halogenierung kann auch in Gegenwart von Katalysatoren, wie Phosphortribromid, Phosphorpentabromid oder Jod, durchgeführt werden. Auch Carbonsäureamide, wie Dimethylformamid oder Benzamid können als Katalysatoren geeignet sein. In bevorzugter Weise ist das erfindungsgemäße Verfahren jedoch ohne diese genannten Katalysatoren durchführbar.

Metalle, wie Eisen oder Aluminium bzw. deren Salze werden vorteilhafterweise aus der Reaktion ausgeschlossen. Es kann hierbei von Vorteil sein, zur Unschädlichmachung kleiner Metallmengen Komplexbildner zuzugeben. Als solche können in Frage kommen: Schwefel, Phosphorpentoxid, Phosphorhalogenide, Hexamethylentetramin, Alkylenpolyamine oder Umsetzungsprodukte von Ammoniak oder Aminen mit Chloressigsäure. Als Komplexbildner oder Katalysatoren verwendete Zusätze können beispielsweise in Mengen von bis zu 10%, bevorzugt 0,1 bis 5%, eingesetzt werden.

Die erfindungsgemäße α-Halogenierung von substituierten Methylaromaten bedeutet einen Ersatz von α-ständigem Wasserstoff in der gegebenenfalls substituierten Methylgruppe, in weiteren vorhandenen gegebenenfalls substituierten Methylgruppen sowie in den gegebenenfalls vorhandenen Alkoxygruppen, soweit diese freie α-Wasserstoffatome tragen, durch Halogen aus dem Bromchlor enthaltenden Halogenierungsgemisch. Für den Fall, daß eine teilweise durch Halogen substituierte Methylgruppe erfindungsgemäß α-halogeniert wird, kann auch ein Halogenaustausch, beispielsweise der Austausch von Chlor gegen Brom, eintreten. Beispielsweise können an der $CHR^1R^2$-Gruppe des erfindungsgemäß einzusetzenden Methylaromaten folgende Reaktionen eintreten, die sinngemäß auch für gegebenenfalls weitere vorhandene $CHR^1R^2$-Gruppen oder für Alkoxygruppen mit freien α-Wasserstoffatomen gelten: für den Fall, daß die Substituenten $R^1$ und $R^2$ ein Halogenatom darstellen, erhält man erfindungsgemäß einen gegebenenfalls substituierten Trihalogenomethylaromaten. Für den Fall, daß nur einer der Substituenten $R^1$ und $R^2$ Halogen bedeutet, kann in Abhängigkeit von der eingesetzten Menge Halogenierungsmittel ein Gemisch erhalten werden, das überwiegend einen Dihalogenmethylaromaten oder überwiegend einen Trihalogenmethylaromaten enthält. Für den Fall, daß sowohl $R^1$ als auch $R^2$ Wasserstoff bedeuten, kann in Abhängigkeit von der eingesetzten Menge des Halogenierungsmittels ein Gemisch erhalten werden, das überwiegend einen Halogenmethylaromaten oder überwiegend einen Dihalogenmethylaromaten oder überwiegend einen Trihalogenmethylaromaten enthält.

Allgemein kann erfindungsgemäß etwa 0,5 bis etwa 3,2 Mol Bromchlor enthaltendes Halogenierungsgemisch pro umzusetzende Methylgruppe eingesetzt werden, wobei diese Werte und die folgenden Angaben sinngemäß für jede weitere gegebenenfalls teilsubstituierte Methylgruppe bzw. jede Alkoxygruppe mit freien α-Wasserstoffatomen zutreffen.

Beispielsweise kann beim Einsatz von etwa 0,5 bis etwa 1,9, bevorzugt 1,2 bis 1,8, besonders bevorzugt 1,5 bis 1,7 Mol Halogenierungsmittel pro Mol gegebenenfalls teilsubstituierte Methylgruppe bevorzugt ein α-ständiges Wasserstoffatom gegen Halogen ausgetauscht werden. Für den Fall, daß Aromaten mit einer nicht-substituierten Methylgruppe eingesetzt werden, enthalten die hierbei erhältlichen Halogenierungsgemische den zugehörigen Trihalogenmethylaromaten in einer Menge von unter 1 Gew.-%, bevorzugt unter 0,1 Gew.-%, bezogen auf die Gesamtmenge der Reaktionsprodukte.

Weiterhin können beim Umsatz von 1,2 bis 1,8, bevorzugt 1,5 bis 1,7, Mol Halogenierungsmittel pro Mol am Aromaten sitzender Methylgruppe Halogenierungsgemische erhalten werden, die etwa 40 bis 85 Gew.-%, bevorzugt 80 bis 85 Gew.-%, Dihalogenmethylaromaten neben etwa 15 bis 60 Gew.-%, bevorzugt 15 bis 20 Gew.-% Halogenmethylaromaten enthalten, wobei die Gewichtsprozente auf die Gesamtmenge der Halogenierungsprodukte bezogen sind.

Weiterhin können beim Einsatz von etwa 1,9 bis etwa 2,2, bevorzugt 2,0 bis 2,1 Mol Halogenierungsmittel pro Mol am Aromaten sitzende Methylgruppe ein Halogenierungsgemisch erhalten werden, das den Dihalogenmethylaromaten in einer Menge von etwa 90 bis 93 Gew.-% enthalten. Bei dieser Variante wird der Halogenmethylaromat und der Trihalogenmethylaromat nur in untergeordneten Mengen von etwa 3 bis 4 Gew.-% bzw. 4 bis 6 Gew.-% gebildet, wobei sich alle

Gew.-%-Angaben wieder auf die Gesamtmenge der Reaktionsprodukte beziehen.

Selbstverständlich können die erhaltenen Halogenierungsgemische, beispielsweise durch Destillation, aufgetrennt werden. Hierbei erhaltene Produktanteile, die entsprechend dem beabsichtigten Halogenierungsgrad noch ungenügend halogeniert sind, können sodann zur Erhöhung der Ausbeute an dem gewünschten Produkt zur weiteren Halogenierung wieder in das Ausgangsreaktionsgemisch zurückführen. Durch diese Verfahrensvariante gelingt es, beispielsweise den Dihalogenmethylaromaten aus einem Aromaten mit nichtsubstituierter Methylgruppe in einer Ausbeute von etwa 95 bis nahe 100 Gew.-% des gesamten durch die Umsetzung erhaltenen Produktgemisches zu erhalten. Daneben kann das Produktgemisch, das durch Einsatz von 1,2 bis 1,8, bevorzugt 1,5 bis 1,7, Mol Halogenierungsgemisch pro Mol Methylgruppe erhalten wurde, noch 0 bis etwa 5 Gew.-%, vorzugsweise weniger als 1 Gew.-%, besonders bevorzugt weniger als 0,1 Gew.-%, an Monohalogenmethylaromat enthalten.

In einer weiteren Variante kann beispielsweise beim Umsatz von etwa 2,2 bis 3,2 Mol, bevorzugt 2,3 bis 2,7 Mol, besonders bevorzugt 2,4 bis 2,5 Mol, Halogenierungsmittel pro Mol nicht-substituierte am Aromaten sitzende Methylgruppe ein Halogenierungsgemisch erhalten werden, das etwa 10 bis 95 Gew.-%, bevorzugt 20 bis 80 Gew.-%, besonders bevorzugt 30 bis 60 Gew.-%, Trihalogenmethylaromaten und etwa 5 bis 90 Gew.-%, bevorzugt 20 bis 80 Gew.-%, besonders bevorzugt 40 bis 70 Gew.-%, Dihalogenmethylaromaten enthält.

Für den Fall, daß die erfindungsgemäß eingesetzten gegebenenfalls substituierten Methylaromaten der Formel (I) Substituenten enthalten, die ebenfalls halogeniert werden können, ist neben den bereits für die Halogenierung von $\alpha$-ständigen Wasserstoffatomen angegebenen Mengen Halogenierungsmittel eine zusätzliche Menge an Halogenierungsmittel zu berücksichtigen, die der jeweils erwarteten Halogenierungsreaktion entspricht und die allgemein 50 bis 150%, bevorzugt 70 bis 130%, besonders bevorzugt 80 bis 120% der stöchiometrisch erforderlichen Menge an Halogenierungsmittel für die jeweilige Reaktion entspricht. Beispiele für solche Halogenierungsreaktionen können neben einer Halogenierung von bereits erwähnten weiteren gegebenenfalls substituierten Methylgruppen und Alkoxygruppen mit $\alpha$-ständigen Wasserstoffatomen beispielsweise noch folgende Reaktionen sein: Die Isothiocyanatgruppe kann zur Isocyaniddihalogenidgruppe reagieren, die Dithiogruppe kann aufgespalten werden und in zwei Cl-S-Gruppen umgewandelt werden.

Die Reaktion des erfindungsgemäßen Verfahrens kann diskontinuierlich oder kontinuierlich durchgeführt werden. Bei diskontinuierlicher Arbeitsweise arbeitet man beispielsweise in einem Rührkessel oder in einer Blasensäule. Bei kontinuierlicher Arbeitsweise sind Schlaufenreaktoren, Blasensäulen, Kaskaden von Blasensäulen, Kaskaden von Rührkesseln geeignet. Bei diskontinuierlicher und kontinuierlicher Reaktionsdurchführung wird dabei darauf geachtet, daß der entstehende Halogenwasserstoff nach Maßgabe seiner Bildung aus dem Reaktionsgemisch entfernt wird. Die Reaktion kann beispielsweise so durchgeführt werden, daß man den zu halogenierenden Methylaromaten auf die gewünschte Temperatur erhitzt und dann unter Konstanthaltung dieser Temperatur oder gegebenenfalls auch unter kontinuierlicher Erhöhung der Reaktionstemperatur das erfindungsgemäß einzusetzende Chlorbrom enthaltende Halogenierungsmittel in den Reaktor einleitet. Die Einleitung des Halogenierungsgemisches kann zweckmäßigerweise unterhalb der Oberfläche des Reaktionsgemisches, besonders zweckmäßig in der unteren Hälfte des Reaktionsgemisches, beispielsweise in der Nähe des Bodens des Reaktionsgefäßes, vorgenommen werden. Dies hat den Vorteil, daß praktisch keine Anteile des Halogenierungsgemisches aus dem Reaktionsgemisch entweichen. Selbstverständlich können etwa doch entweichende Anteile des Halogenierungsgemisches, insbesondere bei rascher Zugabe des Halogenierungsgemisches, aus dem Abgasstrom zurückgewonnen und wieder in die Reaktion eingesetzt werden.

Weiterhin kann der zu halogenierende Methylaromat simultan mit dem Bromchlorid enthaltenden Halogenierungsgemisch in den Reaktor eindosiert werden, wobei die simultane Zugabe sowohl chargenweise als auch kontinuierlich durchgeführt werden kann. Nachdem die vorgesehene Menge an Halogenierungsgemisch zugegeben worden ist, wird bis zum Ende der Halogenwasserstoffentwicklung nachgerührt. Für den Fall, daß das durch Halogenierung erhaltene Gemisch weiter verarbeitet werden soll, ist bei lösungsmittelfreier Arbeitsweise gewöhnlich eine vorherige Aufarbeitung nicht erforderlich. Gegebenenfalls kann es vorteilhaft sein, etwa noch gelösten Halogenwasserstoff im Vakuum vor der direkten Weiterverarbeitung zu entfernen. Bei Bedarf kann jedoch eine weitere Reinigung oder gegebenenfalls eine Fraktionierung des Reaktionsgemisches durch Destillation, Vakuumdestillation oder Umkristallisation erfolgen.

Erfindungsgemäß können Gemische hergestellt werden, die mehr als 50 Gew.-% des Gesamtgewichts des Gemisches an substituierten Methylaromaten der Formel

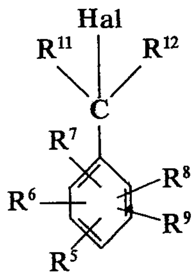

(XIV)

enthalten, in der

Hal für $Br_xCl_{1-x}$ steht, worin x gleich 0,5 bis 0,99 bedeutet,

$R^{11}$ und $R^{12}$ unabhängig voneinander Wasserstoff oder Hal bedeuten, worin Hal die gegebene Bedeutung hat,

$R^8$ für Wasserstoff, Halogen, Nitro, die Sulfonsäuregruppe, Sulfonylhalogenid, die Carboxylgruppe, Alkoxycarbonyl, Aryloxycarbonyl, Carbonylhalogenid, $CHalR^{11}R^{12}$, worin Hal, $R^{11}$ und $R^{12}$ die gegebene Bedeutung haben, Cyano, Isocyanato, Cyanato, Isocyaniddihalogenid, Thiocyanato, Aryl, Aryloxy, Arylthio, die $-S-Cl$-Gruppe, tert.-Alkyl oder gegebenenfalls 1- bis 3fach $\alpha$-halogeniertes Alkoxy steht,

$R^9$ für Wasserstoff, Halogen, Nitro, die Sulfonsäuregruppe, Sulfonylhalogenid, die Carboxylgruppe, Alkoxycarbonyl, Aryloxycarbonyl, Carbonylhalogenid oder $CHalR^{11}R^{12}$, worin Hal, $R^{11}$ und $R^{12}$ die gegebene Bedeutung haben, steht und

$R^5$ bis $R^7$ unabhängig voneinander Wasserstoff oder Halogen bedeuten,

wobei zwei der Reste $R^5$, $R^6$, $R^7$ und $R^9$, wenn sie benachbart sind, Teile eines kondensierten aromatischen oder cycloaliphatischen Ringes sein können und

wobei weiterhin die Arylteile der genannten Substituenten ihrerseits substituiert sein können.

Hal bedeutet das aus dem Halogenierungsemisch in den Methylaromaten eintretende Brom und Chlor, wobei x den Prozentanteil am eintretenden Brom bedeutet. Da die Gesamtmenge an Brom und Chlor an dem durch Hal definierten Ort der Verbindung stets 100% beträgt, ist der Anteil an Chlor entsprechend durch den Ausdruck $1-x$ gegeben. Für x sei beispielsweise ein Wert von 0,5 bis 0,99, bevorzugt 0,6 bis 0,95, besonders bevorzugt 0,7 bis 0,9, das heißt 50 bis 99, bevorzugt 60 bis 95, besonders bevorzugt 70 bis 90 Äquivalent-% des Gesamthalogens angegeben. Selbstverständlich ist diese Betrachtung nicht auf ein einzelnes Molekül anzuwenden, sondern stets auf die Gesamtzahl der im Gemisch anwesenden und durch die den Substituenten Hal enthaltende Formel gekennzeichneten Moleküle anzuwenden. Dies kann beispielsweise bedeuten, daß für den Fall einer Umwandlung einer $CHR^1R^2$-Gruppe in eine $CHalR^{11}R^{12}$-Gruppe x Mol-% als $CBrR^{11}R^{12}$-Gruppe und $(1-x)$ Mol-% als $CClR^{11}R^{12}$-Gruppe vorliegen. Hierbei sei noch einmal auf die bereits oben gemachte Feststellung verwiesen, daß das in der Definition von $R^1$ und $R^2$ enthaltene Chlor oder Brom im erfindungsgemäßen Verfahren der Halogenierung einem Halogenaustausch unterliegen kann, so daß das in den Substituenten $R^{11}$ und $R^{12}$ definitionsgemäß enthaltene Hal ebenfalls unter die angegebenen Definitionen fällt. Beispielsweise kann weiterhin für den Fall, daß $R^{11}$ und $R^{12}$ teilweise Wasserstoff oder Hal bedeuten und aus dieser Definition beispielsweise eine $CHHal_2$-Gruppe vorliegt ein Teil der Moleküle des Gemisches, beispielsweise 10 bis 99 Mol-% mit $CHBr_2$-Gruppe, ein anderer Teil der Moleküle des Gemisches, beispielsweise 1 bis 90 Mol-% mit CHClBr-Gruppe und nur ein kleinerer Teil von beispielsweise bis zu 10 Mol-% als $CHCl_2$-Gruppe vorliegt. Beispielsweise kann weiterhin für den Fall, daß eine $CHal_3$-Gruppe vorliegt, ein Teil der Moleküle des Gemisches, beispielsweise 5 bis 90 Mol-%, mit einer $CBR_3$-Gruppe vorliegen, ein weiterer Teil der Moleküle des Gemisches, beispielsweise 10 bis 90 Mol-% kann mit einer $CBr_2Cl$-Gruppe vorliegen, ein weiterer Teil der Moleküle dieses Gemisches, beispielsweise 5 bis 80 Mol-% kann mit einer $CBrCl_2$-Gruppe vorliegen und nur ein untergeordneter Teil der Moleküle, beispielsweise bis zu 10 Mol-%, kann mit einer $CCl_3$-Gruppe vorliegen.

Die erfindungsgemäß herstellbaren Gemische können weiterhin Ausgangsmaterial, das durch die Formel (I) definiert ist, enthalten, wobei der Anteil des Ausgangsmaterials kleiner als 50 Gew.-% des Gesamtgewichts des Gemisches, bevorzugt kleiner als 10 Gew.-%, besonders bevorzugt kleiner als 1 Gew.-%, beträgt.

Unter den erfindungsgemäß herstellbaren Gemischen seien solche bevorzugt genannt die in der Methylgruppe halogenierten Methylaromaten der Formel

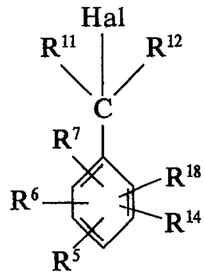

(XV)

enthalten, in der

$R^5$, $R^6$, $R^7$, $R^{11}$, $R^{12}$, $R^{14}$ und Hal die angegebene Bedeutung haben und

$R^{18}$ für Wasserstoff, Halogen, Nitro, die Sulfonsäuregruppe, Sulfonylhalogenid, Alkoxycarbonyl, Phenoxycarbonyl, Carbonylhalogenid, die $CHalR^{11}R^{12}$-Gruppe mit der angegebenen Bedeutung für Hal, $R^{11}$ und $R^{12}$, Phenyl, Phenyloxy, Phenylthio, Phenyldithio, tert.-Alkyl oder gegebenenfalls 1- bis 3fach $\alpha$-halogeniertes Alkoxy steht.

Als gegebenenfalls 1- bis 3fach $\alpha$-halogeniertes Alkoxy sei ein Alkoxy innerhalb des oben gegebenen Bedeutungsumfangs genannt, das für den Fall, daß es 1, 2 oder 3 $\alpha$-Wasserstoffatome trägt, an der $\alpha$-Stelle durch Wasserstoff-Halogen-Austausch 1, 2 oder 3 Hal mit der gegebenen Bedeutung für Hal trägt, wie $OCH_2Hal$, $OCHHal_2$, $OCHal_3$, OCHHal-Alkyl, $OCHal_2$-Alkyl oder $OCHal(Alkyl)_2$, wobei Alkyl oder $(Alkyl)_2$ eine oder zwei geradkettige oder verzweigte Alkylgruppen mit insgesamt weiteren 11 Kohlenstoffatomen im Rahmen der oben gegebenen Definition sein kann. Selbstverständlich kann bei der Zugabe von Halogenierungsmitteln im unteren Bereich des obengenannten Bedeutungsumfanges für die Halogenierungsmittelmenge unverändertes Alkoxy im oben gegebenen Bedeutungsumfang vorliegen.

Als erfindungsgemäß herstellbare Gemische seien weiterhin solche bevorzugt genannt, in denen Verbindungen der Formel

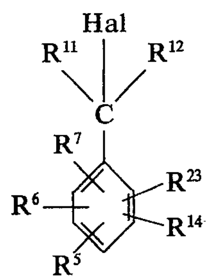

(XVI)

vorliegen, in der

$R^5$, $R^6$, $R^7$, $R^{11}$, $R^{12}$, $R^{14}$, $R^{23}$ und Hal die angegebene Bedeutung haben.

Als erfindungsgemäße herstellbare Gemische seien weiterhin solche bevorzugt genannt, in denen Verbindungen der Formel

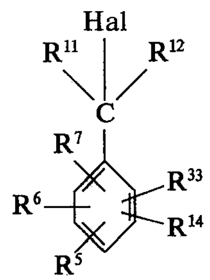

(XVII)

vorliegen, in der

$R^5$, $R^6$, $R^7$, $R^{11}$, $R^{12}$, $R^{14}$, $R^{33}$ und Hal die angegebene Bedeutung haben.

Als erfindungsgemäße herstellbare Gemische seien weiterhin solche bevorzugt genannt, die Verbindungen der Formel

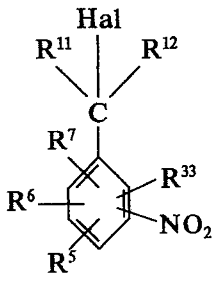

(XVIII)

enthalten, in der

$R^5$, $R^6$, $R^7$, $R^{11}$, $R^{12}$, $R^{23}$ und Hal die angegebene Bedeutung haben.

Bevorzugt sind weiterhin erfindungsgemäß herstellbare Gemische zu nennen, die mehr als 50 Gew.-% des Gesamtgewicht des Gemisches an substituierten, in der Methylgruppe halogenierte Methylaromaten der Formel

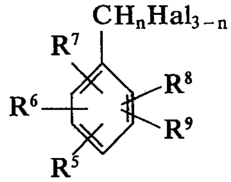

(XIX)

enthalten, in der

$R^5$, $R^6$, $R^7$, $R^8$, $R^9$ und Hal die angegebene Bedeutung haben und
n für die Zahl 0, 1 oder 2 steht.

Unter den zuletzt genannten Gemischen sind wiederum solche bevorzugt zu nennen, die Verbindungen mit Formeln enthalten, die sich von den Formeln (XV), (XVI), (XVII) und (XVIII) in der gleichen Weise ableiten wie die Formel (XIX) von der Formel (XIV).

Besonders bevorzugt zu nennen sind solche Gemische, die 70 bis 95 Gew.-% des Gesamtgewichts des Gemisches an substituierten, in der Methylgruppe halogenierte Methylaromaten der Formel

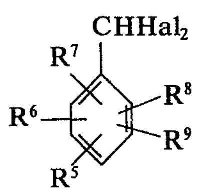

(XX)

enthalten, in der

$R^5$ bis $R^9$ und Hal den angegebenen Bedeutungsumfang haben.

Ganz besonders bevorzugt sind Gemische zu nennen, die mehr als 50 Gew.-% des Gesamtgewichts des Gemisches an Verbindungen der Formeln (XIV) bis (XX) enthalten, bei denen wenigstens einer der Reste $R^8$ und $R^9$ bzw. $R^{14}$ und $R^{18}$ bzw. $R^{14}$ und $R^{23}$ bzw. $R^{14}$ und $R^{33}$ für Nitro steht oder der Rest $R^8$ oder $R^{18}$ für tert.-Alkyl steht.

Bei der bevorzugten erfindungsgemäßen Halogenierung von p-Nitrotoluol verwendet man beispielsweise 1,9 bis 2,2 Mol, vorzugsweise 2,0 bis 2,1 Mol an Bromchlorid enthaltendem Halogenierungsmittel pro Mol p-Nitrotoluol. Man kann dann ein Halogenierungsgemisch erhalten, das mehr als 50 Gew.-%, bevorzugt 70 bis 95 Gew.-%, besonders bevorzugt 80 bis 90 Gew.-%, bezogen auf das Gesamtgewicht des Halogenierungsgemisches an p-Nitrobenzalhalogenid und etwa 5 bis 10 Gew.-% an p-Nitrobenzotrihalogenid enthält. Andererseits ist es möglich, beim Einsatz von etwa 0,5 bis 1,9 Mol an Halogenierungsmittel pro Mol p-Nitrotoluol, Halogenierungsgemische herzustellen, die, bezogen auf umgesetztes p-Nitrotoluol, zu 40 bis 90 Gew.-%, bevorzugt 80 bis 90 Gew.-% aus p-Nitrobenzalhalogenid neben etwa 10 bis 60 Gew.-%, bevorzugt 10 bis 20 Gew.-%, p-Nitrobenzylhalogenid bestehen. Zur Erhöhung der Ausbeute an p-Nitrobenzalhalogenid kann man einen Teil des Halogenierungsgemisches, etwa entsprechend dem Anteil an nicht umgesetztem p-Nitrotoluol und dem Anteil an p-Nitrobenzylhalogenid beispielsweise durch Destillation abtrennen und zur weiteren Halogenierung wieder in das Ausgangsgemisch zurückführen. Durch diese

Verfahrensvariante gelingt es, die Gehalte an p-Nitrobenzalhalogenid auf 90 bis nahe 100 Gew.-%, neben bis zu 5 Gew.-%, vorzugsweise kleiner als 1 Gew.-% insbesondere kleiner als 0,1 Gew.-% an p-Nitrobenzylhalogenid und/oder p-Nitrobenzotrihalogenid in dem Halogenierungsgemisch zu steigern.

Als Halogenid sei hierbei wiederum das Gemisch aus Chlorid und Bromid verstanden, wobei beispielsweise das p-Nitrobenzalhalogenid zum größeren Teil aus p-Nitrobenzalbromid, zum kleineren Teil aus p-Nitrobenzalbromchlorid und zu einem völlig untergeordnetem Maß aus p-Nitrobenzalchlorid besteht. Das Verhältnis von p-Nitrobenzahlbromid zu p-Nitrobenzalbromchlorid beträgt beispielsweise etwa 10 zu 1 bis 2 zu 1, bevorzugt etwa 5 zu 1 bis 3 zu 1, besonders bevorzugt etwa 4 zu 1.

Ganz besonders bevorzugt zu nennen sind demnach Gemische, die mehr als 50 Gew.-% des Gesamtgewichts des Gemisches an in der Methylgruppe halogenierten p-$NO_2$-Toluolen der Formel

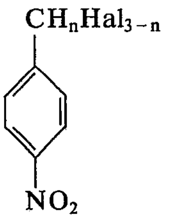

(XXI)

enthalten, in der

Hal und n den oben gegebenen Bedeutungsumfang haben und die gegebenenfalls noch p-Nitrotoluol, mit einem Anteil von kleiner als 50 Gew.-%, bevorzugt kleiner als 10 Gew.-%, besonders bevorzugt kleiner als 1 Gew.-% des Gesamtgewichts des Gemisches enthalten.

In ganz besonders bevorzugter Weise seien Gemische genannt, die 70 bis 95 Gew.-% des Gesamtgewichts des Gemisches an in der Methylgruppe substituierten p-$NO_2$-Toluolen der Formel

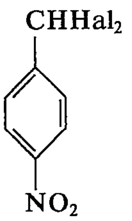

(XXII)

enthalten, in der

Hal den angegebenen Bedeutungsumfang hat.

Die erfindungsgemäß durch Halogenierung erhältlichen Gemische können beispielsweise zur Herstellung der entsprechenden Alkohole, Aldehyde, Carbonsäurehalogenide und Carbonsäuren verwendet werden. Beispielsweise werden die in den erfindungsgemäß erhältlichen Halogenierungsgemischen enthaltenden Halogenmethyl-Aromaten zur Herstellung von Benzylalkoholen verwendet. Die erfindungsgemäß hergestellten Dihalogenmethyl-Aromaten können zur Herstellung der zugehörigen Aldehyde dienen. Die erfindungsgemäß hergestellten Trihalogenmethyl-Aromaten dienen beispielsweise zur Herstellung von Säurehalogeniden oder zur Herstellung der entsprechenden Carbonsäuren.

Die erhältlichen Benzylhalogenide bzw. die zugehörigen Benzylalkohole und die Benzalhalogenide bzw. die zugehörigen Benzaldehyde sind wertvolle Ausgangsprodukte für die Herstellung von Riechstoffen, Pharmazeutika und Schädlingsbekämpfungsstoffen. Die durch Verseifung der Benzotrihalogenide erhältlichen Benzoesäuren oder Benzoylhalogenide finden Verwendung in speziellen Lackrohstoffen und Alkydharzen. Nitrobenzaldehyde, die aus den zugehörigen Benzalhalogeniden durch Verseifung zugänglich sind, sind beispielsweise Zwischenprodukte für Farbstoffe der verschiedensten Klassen (Ullmanns Enzyklopädie der Technischen Chemie, 3. Auflage, 4. Band, Seite 241, Verlag Urban und Schwarzenberg, Berlin-München 1953). Die Verseifung der Benzalhalogenide zu den zugehörigen Benzaldehyden kann beispielsweise durch 60—90 gew.-%ige Schwefelsäure oder Ameisensäure oder durch Erhitzen unter Druck in Gegenwart von Wasser und Salzen schwacher Säuren erfolgen.

Das erfindungsgemäße Verfahren verläuft in bezug auf die Selektivität der Herstellung von Benzalhalogeniden und Benzylhalogeniden der Seitenkettenbromierung vergleichbar. Nebenreaktionen wie beispielsweise die Substitution $\beta$-ständiger Wasserstoffatome in tert.-Alkylgruppen oder der Austausch von Kernsubstituenten wie Nitro, Sulfo, Sulfochlorid oder Carbonylchlorid werden nur in einem vernachlässigbaren Maße beobachtet. Durch die erfindungsgemäße Halogenierung unter

Verwendung von Chlorbrom als Halogenierungsmittel ist es daher möglich, gegenüber der nach dem Stand der Technik erfolgenden Bromierung einen Teil des Broms durch Chlor zu ersetzen, ohne die Vorteile der Seitenkettenbromierung zu verlieren.

Es ist überraschend, daß eine polar aufgebaute Verbindung wie Chlorbrom für eine Seitenketten-Bromierung eingesetzt werden kann. Aufgrund des polaren Aufbaus konnte vielmehr angenommen werden, daß etwa eine Kernhalogenierung stärker in Erscheinung treten müßte.

Beispiel 1

In einem 500-ml-Dreihalskolben mit Topftrichter, dessen Einleitungsrohr bis an den Kolbenboden reicht, werden 109,6 g (0,8 Mol) 4-Nitrotoluol unter Rühren auf 150° C aufgeheizt. Im Verlauf von ca. 5 Stunden werden 174,0 g (1,5 Mol) Bromchlorid so zugetropft, daß im Rückflußkühler kein Bromchlorid erscheint. Es wird noch ca. 10 Minuten bis zum Ende der Gasentwicklung nachgerührt. Beim Abkühlen erstarrt das Reaktionsgemisch zu ca. 210 g einer zwischen 50—60° C schmelzenden Kristallmasse, die zu 80—85° C aus 4-Nitrobenzalhalogenid, etwa 10% aus 4-Nitrobenzylhalogenid und zu etwa 5% aus 4-Nitrobenzotrihalogenid besteht.

Beispiel 2 (zur Verwendung)

In einem 500-ml-Vierhalskolben mit Rührer, Rückflußkühler, Thermometer, Kapillare und Anschluß an die Wasserstrahlpumpe wird folgender Ansatz verarbeitet

40,0 g (0,132 mol; ca. 97%ig) p-Nitrobenzalhalogenid
320 g 96%ige Schwefelsäure
400 g Eis

Benzalhalogenid und Schwefelsäure werden vorgelegt und unter Rühren wird bei voll angelegtem Wasserstrahlvakuum durch die Kapillare Stickstoff eingeleitet. Dann wird das Gemisch auf 90° C aufgeheizt, wobei das Benzalhalogenid in Lösung geht. Das Vakuum im Reaktionskolben beträgt 150—200 mbar.

Nach 10 Stunden ist alles Benzalhalogenid umgesetzt. Die rotbraune Lösung wird nach dem Abkühlen auf Eis geschüttet, 30 Minuten verrührt, abgesaugt, neutral gewaschen und bei 50° C im Vakuum getrocknet.

Es werden 19,0 g eines beigefarbenen Produktes erhalten (Fp.: 101—103° C) das folgende Komponenten enthält:

92,9% p-Nitrobenzaldehyd
3,6% p-Nitrobenzoesäure

Dies entspricht einer Ausbeute von ca. 95% der theoretischen Ausbeute.

Beispiel 3 (Zur Verwendung)

In einem 0,7-Liter-V4A-Stahlautoklav mit Rührer (400 UPM) wird folgender Ansatz verarbeitet:

40,0 g (0,132 mol; ca. 97%ig) p-Nitrobenzalhalogenid
170 g Wasser
30 g (0,3 Mol) $CaCO_3$

In den mit Stickstoff gespülten Autoklaven wird die Aufschlämmung von Benzalhalogenid und Schlämmkreide in Wasser unter Rühren auf 150° C erhitzt. Durch freigesetztes $CO_2$ entsteht dabei ein Druck von 13,5 bar. Nach 5 Stunden wird der Autoklav abgekühlt, entspannt und ausgenommen. Es wird abgesaugt und der Filterkuchen zweimal mit jeweils 100 ml heißem Ethanol extrahiert. Nach dem Abdampfen des Ethanol am Rotationsverdampfer und dem Trocknen des Rückstands bei 50° C im Vakuum bleiben 21,0 g eines gelblichen Produktes (Fp.: 103—104° C), das folgende Komponenten enthält:

93,4% p-Nitrobenzaldehyd
0,4—0,5% p-Nitrobenzoesäure

Dies entspricht einer Ausbeute von ca. 98% der theoretischen Ausbeute.

**Patentansprüche**

1. Verfahren zur α-Halogenierung von gegebenenfalls substituierten Methylaromaten, dadurch gekennzeichnet, daß man Methylaromaten der allgemeinen Formel

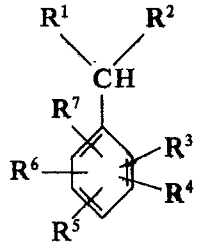

in der

$R^1$ und $R^2$ unabhängig voneinander Wasserstoff, Chlor oder Brom bedeuten,
$R^3$ für Wasserstoff, Halogen, Nitro, die Sulfonsäuregruppe, Sulfonylhalogenid, die Carboxylgruppe, Alkoxycarbonyl, Aryloxycarbonyl, Carbonylhalogenid, die $CHR^1R^2$-Gruppe, wobei $R^1$ und $R^2$ die gegebene Bedeutung haben, Cyano, Isocyanato, Cyanato, Isothiocyanato, Thiocyanato, Aryl, Aryloxy, Arylthio, Aryldithio, tert.-Alkyl oder Alkoxy steht,
$R^4$ für Wasserstoff, Halogen, Nitro, die Sulfonsäuregruppe, Sulfonylhalogenid, die Carboxylgruppe, Alkoxycarbonyl, Aryloxycarbonyl, Carbonylhalogenid oder die $CHR^1R^2$-Gruppe, worin $R^1$ und $R^2$ die gegebene Bedeutung haben, steht und
$R^5$ bis $R^7$ unabhängig voneinander Wasserstoff oder Halogen bedeuten,

wobei zwei der Reste $R^4$, $R^5$, $R^6$ und $R^7$, wenn sie benachbart sind, Teile eines kondensierten aromatischen oder cycloaliphatischen Ringes sein können und
wobei weiterhin die Arylteile der genannten Substituenten ihrerseits substituiert sein können, mit Ausnahme von gegebenenfalls substituierten 4-tert.-Butyltoluolen, bei einer Temperatur von 40 bis 250°C mit Chlorbrom oder Chlorbrom enthaltenden Halogenierungsmitteln umsetzt, wobei gegebenenfalls in Gegenwart von Radikalbildnern und/oder bei Bestrahlung mit Licht sowie gegebenenfalls in Gegenwart von Lösungsmitteln gearbeitet wird.

2. Verfahren nach Anspruch 1, dadurch gekennzeichnet, daß man 0,5 bis 3,2 Mol, gerechnet als Bromchlorid, an Halogenierungsmittel pro Mol Methylaromaten einsetzt.

3. Verfahren nach Anspruch 1, dadurch gekennzeichnet, daß man 0,5 bis 1,9 Mol Halogenierungsmittel einsetzt.

4. Verfahren nach Anspruch 1, dadurch gekennzeichnet, daß man 1,9 bis 2,2 Mol Halogenierungsmittel einsetzt.

5. Verfahren nach Anspruch 1, dadurch gekennzeichnet, daß man 2,2 bis 3,2 Mol Halogenierungsmittel einsetzt.

6. Verfahren nach Ansprüchen 1 bis 5, dadurch gekennzeichnet, daß man die Umsetzung bei einer Temperatur von 80 bis 220°C durchführt.

**Claims**

1. Process for the α-halogenation of optionally substituted methyl-aromatics, characterised in that methyl-aromatics of the general formula

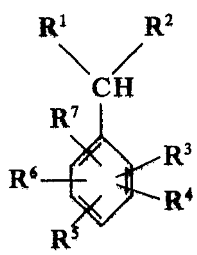

in which

$R^1$ and $R^2$ independently of one another denote hydrogen, chlorine or bromine,
$R^3$ represents hydrogen, halogen, nitro, the sulphonic acid group, sulphonyl halide, the carboxyl group, alkoxycarbonyl, aryloxycarbonyl, carbonyl halide or the $CHR^1R^2$ group,

wherein

$R^1$ and $R^2$ have the meaning given, or

$R^3$ represents cyano, isocyanato, cyanato, isothiocyanato, thiocyanato, aryl, aryloxy, arylthio, aryldithio, tert.-alkyl or alkoxy,

$R^4$ represents hydrogen, halogen, nitro, the sulphonic acid group, sulphonyl halide, the carboxyl group, alkoxycarbonyl, aryloxycarbonyl, carbonyl halide or the $CHR^1R^2$ group,

wherein

$R^1$ and $R^2$ have the meaning given, and

$R^5$ to $R^7$ independently of one another denote hydrogen or halogen,

and wherein

two of the radicals $R^4$, $R^5$, $R^6$ and $R^7$, if they are adjacent, can be members of a fused-on aromatic or cycloaliphatic ring,

and, wherein, furthermore, the aryl parts of the substituents mentioned can in turn be substituted, with the exception of optionally substituted 4-tert.-butyl toluenes, are reacted with chlorobromine or halogenating agents containing chlorobromine at a temperature of 40 to 250°C, the reaction being carried out, if appropriate, in the presence of agents which form free radicals and/or under irradiation with light, and, if appropriate, in the presence of solvents.

2. Process according to Claim 1, characterised in that 0.5 to 3.2 mols, calculated as bromine chloride, of halogenating agent are employed per mol of methyl-aromatic.

3. Process according to Claim 1, characterised in that 0.5 to 1.9 mols of halogenating agent are employed.

4. Process according to Claim 1, characterised in that 1.9 to 2.2 mols of halogenating agent are employed.

5. Process according to Claim 1, characterised in that 2.2 to 3.2 mols of halogenating agent are employed.

6. Process according to Claims 1 to 5, characterised in that the reaction is carried out at a temperature of 80 to 220°C.

**Revendications**

1. Procédé d'$\alpha$-halogénation de composés méthylaromatiques éventuellement substitués, caractérisé en ce qu'on fait réagir des composés méthylaromatiques de formule générale

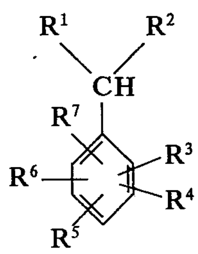

dans laquelle

$R^1$ et $R^2$ désignent, indépendamment l'un de l'autre, l'hydrogène, le chlore ou le brome,

$R^3$ représente l'hydrogène, un halogène, un groupe nitro, le groupe acide sulfonique, un groupe halogénure de sulfonyle, le groupe carboxyle, un groupe alkoxycarbonyle, aryloxycarbonyle, halogénure de carbonyle, le groupe $CHR^1R^2$ dans lequel $R^1$ et $R^2$ ont la définition indiquée, un groupe cyano, isocyanato, cyanato, isothiocyanato, thiocyanato, aryle, aryloxy, arylthio, aryldithio, tertio-alkyle ou alkoxy,

$R^4$ représente l'hydrogène, un halogène, un groupe nitro, le groupe acide sulfonique, un groupe halogénure de sulfonyle, le groupe carboxyle, un groupe alkoxycarbonyle, aryloxycarbonyle, halogénure de carbonyle ou le groupe $CHR^1R^2$ dans lequel $R^1$ et $R^2$ ont la définition indiquée ci-dessus, et

$R^5$ à $R^7$ désignent, indépendamment l'un de l'autre, l'hydrogène ou un halogène,

deux des restes $R^4$, $R^5$, $R^6$ et $R^7$, lorsqu'ils sont voisins, pouvant appartenir à un noyau aromatique ou cycloaliphatique condensé et les parties aryle des substituants mentionnés pouvant, en outre, être substituées elles aussi, à l'exception de 4-tertio-butyl-toluènes éventuellement substitués, à une température de 40 à 250°C avec le bromure de chlore ou des agents d'halogénation contenant du bromure de chlore, et on opère alors, le cas échéant, en présence de générateurs de radicaux et/ou par irradiation avec de la lumière ainsi que, le cas échéant, en présence de solvants.

2. Procédé suivant la revendication 1, caractérisé en ce qu'on utilise 0,5 à 3,2 moles, exprimées en chlorure de brome, d'agent d'halogénation par mole de composés méthylaromatiques.

3. Procédé suivant la revendication 1, caractérisé en ce qu'on utilise 0,5 à 1,9 mole d'agent d'halogénation.

4. Procédé suivant la revendication 1, caractérisé en ce qu'on utilise 1,9 à 2,2 moles d'agent d'halogénation.

5. Procédé suivant la revendication 1, caractérisé en ce qu'on utilise 2,2 à 3,2 moles d'agent d'halogénation.

6. Procédé suivant les revendications 1 à 5, caractérisé en ce qu'on conduit la réaction à une température de 80 à 220° C.